# EUROPÄISCHE PATENTSCHRIFT

(11) **EP 2 226 031 B1**
(45) Veröffentlichungstag und Bekanntmachung des Hinweises auf die Patenterteilung: **07.09.2011**
(21) Anmeldenummer: 10002220.1
(22) Anmeldetag: 04.03.2010
(51) Int. Cl.: A61B 18/22, A61B 17/3211, A61B 18/24

(54) **Laserskalpell**
Laser scalpel
Scalpel laser

(30) Priorität: 06.03.2009 DE 102009011587
(43) Veröffentlichungstag der Anmeldung: 08.09.2010
(73) Patentinhaber: A.R.C. Laser GmbH, 90411 Nürnberg (DE)
(72) Erfinder: Thyzel, Reinhold, 90562 Heroldsberg (DE); Volland, Prof. Dr. Gerd, 91058 Erlangen-Bruck (DE); Walker, Dr. Rudolf, 91077 Neunkirchen am Brand (DE)
(74) Vertreter: Schröer, Gernot H.

(56) Entgegenhaltungen:
- EP-A2- 0 453 320
- WO-A1-01/87176
- WO-A1-03/009767
- US-A- 4 273 127

## Beschreibung

Die Erfindung betrifft ein Laserskalpell.

Aus WO 01/87176 A1 ist ein chirurgisches Laserskalpell bekannt mit einem aus einem Diamantkristall gebildeten Skalpellkörper, der eine geschliffene Skalpellklinge und geschliffene Außenflächen aufweist, einerseits und einem Laser, dessen Laserlicht über eine Lichtleitfaser in den Skalpellkörper eingeleitet wird und überwiegend im Bereich der Skalpellklinge wieder aus dem Skalpellkörper austritt, andererseits. Die austretende Laserstrahlung im Bereich der Skalpellklinge bewirkt eine Photokoagulation des Blutes und damit eine Reduzierung der Blutung der Wunde. In einer in FIG 10a und 10b beschriebenen Ausführungsform ist die Laserachse des Lichtleiters an einer Einkoppelfläche an einer flachen Kristallaußenfläche senkrecht zur entsprechenden Kristallaußenfläche gerichtet und trifft der Laserstrahl somit senkrecht auf den Skalpellkörper auf und liegt ferner die Skalpellschneide im wesentlichen unter einem rechten Winkel seitlich versetzt zur Einkoppelfläche. Das eingekoppelte Laserlicht verläuft in dem Skalpellkörper bis zu einer Reflexionsaußenfläche gegenüber der Einkoppelfläche, wird dort intern reflektiert und tritt dann an der Skalpellschneide aus dem Skalpellkörper aus. Die Hauptausbreitungsrichtung oder optische Achse des Laserlichts wird an der Reflexionsfläche jeweils etwa um 90° geändert, so dass der Einfallswinkel und Ausfallswinkel an der Reflexionsfläche für den zentralen Strahl des Laserlichts 45° beträgt. Gemäß FIG 10a trifft der Laserstrahl auf eine nach außen konkav und nach innen konvex gekrümmte Reflexionsfläche und wird deshalb dort aufgeweitet. Wählt man die Reflexionsfläche nach außen konvex und nach innen konkav, so kann der Laserstrahlfokus an der Austrittsfläche an der Skalpellschneide fokussiert werden. In FIG 10b wird die ansonsten flache Kristallaußenfläche im Bereich der Einkoppelfläche nach außen konkav und nach Innen konvex gebildet und bildet somit eine Art Linse an der Oberfläche des Skalpellkörpers, so dass der ursprünglich parallele Laserstrahl aufgrund der Brechung dieser Linse aufgeweitet wird und der aufgeweitete Laserstrahl dann auf die flache Reflexionsfläche an der anderen Seite des Skalpellkörperkristalls trifft und von dort dann zur Laserschneide reflektiert wird. Auch in FIG 10b kann durch eine umgekehrte Ausgestaltung der konkaven, nach innen konvexen Eintrittsfläche in eine nach außen konvexe und nach innen konkave Oberfläche nun der Laserstrahl fokussiert werden und trifft dann als fokussierter Laserstrahl auf die Reflexionsfläche. Die Ausbildung einer konkaven oder konvexen Einkoppelfläche, die wie eine Linse wirkt, an dem Kristall und die genau darauf zu erfolgende Justierung des Laserlichts ist vergleichsweise aufwändig. Die konkave oder konvexe Ausbildung der Reflexionsfläche gemäß FIG 10a bedeutet ebenfalls einen erhöhten Aufwand beim Schleifen des Kristalls.

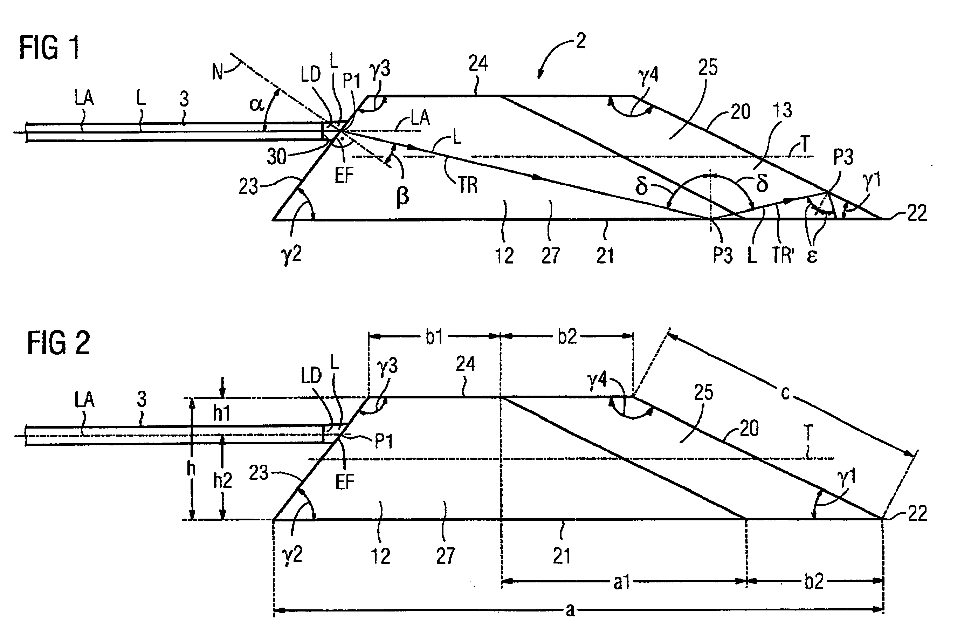

Die WO 03/009767 A1 offenbart ein Laserskalpell mit einem Skalpellkörper, der aus einem für Laserstrahlung wenigstens eines bestimmten Wellenlängenbereichs transmissivem Material besteht und gemäß FIG 1 eine Spitze und zwei Schneidkanten sowie einen Skalpellrücken aufweist, wobei die Skalpelischneiden und der Skalpellrücken in der Spitze zusammenlaufen. An der von den Skalpellschneiden abgewandten Rückseite ist eine Einkoppelfläche für aus dem Ende eines Lichtleiters austretendes Laserlicht, das unter senkrechtere Inzidenz eintritt und sich durch den Skalpellkörper über interne Reflektionen bis zu der Spitze und den Schneidkanten fortpflanzt und dort zur Koagulation verwendet wird. Es wird die zurückkommende Strahlung auf demselben Weg wieder zurück in den Lichtleiter geleitet und für Messungen ausgewertet.

Die US 4,273,127 offenbart ein Laserskalpell mit einem für die Laserstrahlung transmissivem Skalpellkörper, der eine Skalpellschneide aufweist. In diesen Skalpellkörper wird über einen Lichtwellenleiter Laserlicht eingekoppelt, das sich bis zur Skalpellschneide innerhalb des Skalpellkörpers fortpflanzt und dort wieder austritt. Das Laserlicht wird an der Schneide zur Koagulation verwendet.

Der Erfindung liegt die Aufgabe zugrunde, ein Laserskalpell anzugeben, mit dem ein hoher Anteil des in den Skalpellkörper eingekoppelten Laserlichts an der Skalpellschneide zur Verfügung gestellt werden kann oder austritt und das dennoch vergleichsweise einfach herzustellen ist.

Diese Aufgabe wird gemäß der Erfindung gelöst mit den Merkmalen des Patentanspruchs 1. Vorteilhafte Ausgestaltung und Weiterbildungen gemäß der Erfindung ergeben sich aus den abhängigen Patentansprüchen.

Das Laserskalpell gemäß Patentanspruch 1 umfasst einen Skalpellkörper aus einem für Laserstrahlung wenigstens eines bestimmten Wellenlängenbereiches transmissivem Material, wobei der Skalpellkörper eine Skalpellschneide und einen Skalpellrücken aufweist und die Skalpellschneide und der Skalpellrücken in einer Spitze zusammenlaufen und wobei der Skalpellkörper ferner an einer von der Skalpellschneide abgewandten Rückseite eine Einkoppelfläche zum Einkoppeln von Laserstrahlung aufweist.

Die Erfindung beruht zunächst auf der Überlegung, das Laserlicht (oder: den Laserstrahl) unter einem zur Flächennormalen (oder: Einfall- oder Inzidenzachse) der Einkoppelfläche am Skalpellkörper, also der senkrecht oder normal zur Einkoppelfläche gerichteten Richtung oder Achse, spitzen Einkoppelwinkel aus einem Winkelbereich zwischen 25° und 45° in den Skalpellkörper einzukoppeln und nicht in senkrechter Inzidenz. Dadurch wird der Laserstrahl zur Flächennormalen hin gebrochen und verläuft dann mehr in die Richtung oder näher zur Spitze des Skalpellkörpers hin als vor dem Eintritt in den Skalpellkörper. Ferner ist die Einkoppelfläche am Skalpellkörper so angeordnet und dimensioniert, dass ein Teil der an der Einkoppelfläche eingekoppelten Laserstrahlung an dem Skalpellrücken wenigstens teilweise durch interne Reflexion zur Skalpellschneide hin reflektiert wird und ein weiterer Teil der an der Einkoppelfläche eingekoppelten Laserstrahlung sich auf direktem Weg durch den Skalpellkörper zu dessen Skalpellschneide oder dessen Spitze fortpflanzt. Schließlich weist die Einkoppelfläche von dem Skalpellrücken einen größeren Abstand auf als von einer Bodenfläche des Skalpellkörpers, welche die Skalpellschneide an dem von der Spitze abgewandten Ende mit der Rückseite des Skalpellkörpers verbindet.

Die Erfindung wird im Folgenden anhand von Ausführungsbeispielen weiter erläutert. Dabei wird auch auf die Zeichnungen Bezug genommen, in deren
- FIG 1: ein Laserskalpell mit einem Skalpellkörper und einem Licht- leiter in einer Seitenansicht,
- FIG 2: der Skalpellkörper gemäß FIG 1 mit Bemaßungen in einer Seitenansicht,
- FIG 3: der Skalpellkörper mit Lichtleitfaser gemäß FIG 1 in einer Draufsicht von oben und
- FIG 4: eine zusätzlich angeschliffene Spitze eines Skalpellkörpers, insbesondere gemäß FIG 1 bis 3, in einer Draufsicht von oben

jeweils schematisch dargestellt sind. Einander entsprechende Teile und Größen sind in den FIG 1 bis 4 mit denselben Bezugszeichen versehen, soweit sich nicht aus der Figurenbeschreibung etwas anderes ergibt.

Das Laserskalpell (oder: Laserklinge, Lasermesser) 2 gemäß FIG 1 bis 3 umfasst einen Skalpellkörper 12 und einen Lichtleiter 3, insbesondere eine Lichtleitfaser, zum Einkoppeln von Laserlicht (oder Laserstrahlung, Laserstrahl) L einer nicht dargestellten Laserquelle in den Skalpellkörper 12.

Der Skalpellkörper 12 ist im Wesentlichen als unregelmäßiger Polyeder ausgebildet mit mehreren ebenen oder flachen Polyederflächen (oder: Flachseiten, Außenflächen, Facetten), die gemeinsame Kanten miteinander bilden und den Skalpellkörper 12 nach außen begrenzen. Als Polyederflächen des Skalpellkörpers 12 sind ein Skalpellrücken 21, eine Rückseite 23, eine Bodenfläche 24, zwei Seitenflächen 27 und 28 und schließlich zwei Schneidenflächen 25 und 26, die in einer Schneidkante 20 zusammenlaufen, vorgesehen. Bezüglich einer Mittelebene M ist der Skalpellkörper 12 spiegelsymmetrisch ausgebildet. Die Bodenfläche 24 und der Skalpellrücken 21 verlaufen parallel zueinander unter einem konstanten Abstand, der die Höhe h des Skalpellkörpers 12 definiert.

Die an der Vorderseite 13 des Skalpellkörpers 12 gebildete Skalpellschneide umfasst die Schneidkante 20 und die beiden zueinander geneigten Schneidenflächen 25 und 26, die sich einerseits an ihren Längsseiten in der Schneidkante 20 treffen. Die Schneidenflächen 25 und 26 bilden ferner jeweils eine kürzere gemeinsame Kante sowohl mit dem Skalpellrücken 21 als auch mit der Bodenfläche 24. Die Skalpellschneide, also deren Schneidflächen 25 und 26 sowie deren Schneidkante 20, trifft bzw. treffen sich (oder: laufen zusammen) mit dem Skalpellrücken 21 in einer Spitze 22 des Skalpellkörpers 12, die zudem zentral in der Mittelebene M liegt. Mit der Schneidkante 20 bildet der Skalpellrücken 21 an der Spitze 22 den spitzen Innenwinkel γ1, der insbesondere zwischen 21° und 35°, vorzugsweise zwischen 25° und 30° gewählt ist, beispielsweise bei etwa 28°, so dass die Schneidkante 20 auf den Schneidrücken 21 zu nach innen geneigt ist.

Ferner haben die Schneidenflächen 25 und 26 die konstante, in Längsrichtung gemessene Abmessung b2 über die gesamte Höhe h, haben also jeweils die Form eines Parallelogramms, das um die Länge a1 parallel verschoben ist. Die Länge c der Schneidkante 20 ergibt sich aufgrund geometrischer Beziehungen als c = a1/cos γ1 = h/sin γ1. Die Länge b1 der Bodenfläche 24 bis zum Beginn der schrägen, angeschliffenen Schneidenflächen 25 und 26 ist mit b1 bezeichnet und die weitere Länge der Bodenfläche 24, die dann aufgrund der schräg nach innen verlaufenden Schneldenflächen 25 und 26 die Form eines gleichschenkligen Dreiecks hat, wie in FIG 2 zu sehen, mit b2.

Die Schneidenflächen 25 und 26 der Skalpellschneide und ihre gemeinsamen Kanten mit dem Skalpellrücken 21 sind unter einem spitzen Innenwinkel von γ5, der beispielsweise 30° beträgt, zur Mittelebene M gerichtet und somit nach innen auf die Mittelebene M zu und aufeinander zu geneigt oder schräg gestellt. Dadurch schließen die Schneidenflächen den doppelten Innenwinkel 2 γ5 miteinander ein, der zugleich den Keilwinkel oder Innenwinkel der Skalpellschneide bildet.

Ferner verbindet der Skalpellrücken 21 die Spitze 22 der Schneidkante 20 mit der ebenfalls als Flachseite oder ebene Fläche ausgebildeten Rückseite 23 und bildet mit der Rückseite 23 an der gemeinsamen Kante den spitzen Innenwinkel γ2, d.h. die Rückseite 23 ist schräg zum Skalpellrücken 21 hin geneigt. Die Rückseite 23 ist an ihrer Oberseite über die Bodenfläche 24 mit der Skalpellschneide verbunden und bildet mit der Bodenfläche 24 an der gemeinsamen Kante den stumpfen Innenwinkel γ3. Die Bodenfläche 24 bildet wiederum mit der Schneidkante 20 an dem gemeinsamen Eckpunkt den stumpfen Innenwinkel γ4. In der Mittelebene M des Skalpellkörpers 12 bildet somit der Skalpellkörper 12 das in FIG 1 zu sehende Trapez mit den Innenwinkel γ1, γ2, γ3 und γ4 und den Seitenkanten, die durch die Schneidkante 20, den Skalpellrücken 21, die Rückseite 23 und die Bodenfläche 24 gebildet sind. Es gilt γ3= 180° - γ2 und γ1 + γ4 = 180°, da die Bodenfläche 24 und der Skalpellrücken 21 parallel zueinander verlaufende Flächen sind.

Der Skalpellrücken 21 ist mit der Bodenfläche 24 im hinteren Bereich über die zwei Seitenflächen 27 und 28 und im vorderen Bereich über die Schneidenflächen 25 und 26 verbunden. Die beiden Seitenflächen 27 und 28 verlaufen parallel zueinander unter dem Abstand d, der der Breite des Skalpellkörpers 12 senkrecht zu dessen Mittelebene M entspricht, und sind parallel zur Mittelebene M und orthogonal zum Skalpellrücken 21 gerichtet.

Der Skalpellkörper 12 ist nun aus einem Material gebildet, das zumindest in einem vorgegebenen Transmissionsspektrum oder -wellenlängenbereich für das Laserlicht L transmissiv oder transparent ist und andererseits die erforderlichen mechanischen Eigenschaften aufweist, um die Skalpellschneide auszubilden und als chirurgisches oder mechanisches Skalpell oder Klinge dienen zu können. Insbesondere muss das Material ausreichend hart und stabil sein und auch gut schärfbar oder schleifbar sein. Ein bevorzugtes Material ist ein kristallines, vorzugsweise einkristallines, Material wie beispielsweise Saphir oder Diamant.

Das Transmissionsspektrum des Skalpellkörpers 12 liegt im Allgemeinen innerhalb eines Wellenlängenbereiches von 380 nm bis 1200 nm, der den sichtbaren Spektralbereich und einen Teilbereich des Infrarotbereiches umfasst. Die Wellenlänge oder das Wellenlängenspektrum des Laserlichts L liegt innerhalb des Transmissionssektrums des Skalpellkörpers 12, damit dieser das Laserlicht L passieren lässt.

Der Skalpellkörper 12 ist insbesondere durch Anschleifen eines Kristalls erzeugt, indem einzelne Flachseiten oder Facetten in den Kristall geschliffen werden. Selbstverständlich ist die Form der Schneidkante 20 als exakt geradlinige Schneide idealisiert. Mikroskopisch betrachtet weicht eine geschliffene Schneidkante 20 von der idealen geometrischen linearen Gestalt ab und kann mikroskopisch kleine Unregelmäßigkeiten wie Wellen etc. aufweisen. Es genügt hier, wenn im Verhältnis zu dem hier zu schneidenden menschlichen oder tierischen Körpergewebe die Schneidkante 20 im Wesentlichen glatt und kontinuierlich ist und damit auch als linear anzusehen ist.

Der Skalpellkörper 12 hat also die Form eines chirurgischen Skalpells oder einer Klinge oder eines Messers, das für das Operieren oder chirurgische Arbeiten an Menschen oder Tieren geeignet ist, wobei mit der Skalpellschneide geschnitten wird.

Das Laserlicht L wird zum Koagulieren oder Gerinnen des beim Schneiden austretenden Blutes und damit zum Verschließen der Wunde oder des Gewebes *in situ* verwendet. Dazu soll möglichst ein großer Anteil des Laserlichts L, das aus dem Ende 30 des Lichtleiters 3 austritt, durch den Skalpellkörper 12 in den Bereich der Skalpellschneide gelangen, vorzugsweise wiederum ein möglichst großer Anteil davon in der Nähe der Spitze 22. Eine Einkoppelfläche EF zum Einkoppeln des Laserlichts L aus dem Lichtleiter 3 in den Skalpellkörper 12 ist an dessen Rückseite 23 vorgesehen.

Der Skalpellkörper 12 wird im Allgemeinen an einem nicht dargestellten Handhalter befestigt, den der Chirurg in die Hand nimmt während der Operation. An diesem Handhalter wird auch die Lichtfaser oder der Lichtleiter 3 befestigt und bezüglich der Einkoppelfläche EF an der Rückseite 23 des Skalpellkörpers 12 optisch justiert und fixiert.

Die optische Achse des Lichtleiters 3 bildet eine Hauptausbreitungsrichtung des Laserlichts L, entlang der sich das Laserlicht L im Lichtleiter 3 fortpflanzt, und ist als Laserachse LA bezeichnet. Die Laserachse LA ist parallel zum Skalpellrücken 21 gerichtet und verläuft bevorzugt auch in der Mittelebene M des Skalpellkörpers 12. Beim Austritt des Laserlichts L aus dem von der Rückseite 23 des Skalpellkörpers 12 beabstandeten Ende 30 des Lichtleiters 3 weitet sich der Laserstrahl in einem Laserdivergenzbereich LD gemäß einer durch die numerische Apertur des Lichtleiters 3 quantifizierbaren Divergenz auf. In diesem Lichtdivergenzbereich LD pflanzt sich das Laserlicht L frei durch die Luft (oder ein anders Gas) fort und trifft dann auf die Oberfläche der schrägen Rückseite 23 des Skalpellkörpers 12 in dem Teilbereich, der die Einkoppelfläche EF bildet. Da die Rückseite 23 des Skalpellkörpers 12 flach oder eben ausgebildet ist, liegt auch die Einkoppelfläche EF im Wesentlichen in einer Ebene, die schräg zur Laserachse LA gerichtet ist. Beispielsweise ist bei einem im Wesentlichen kreisförmigen Querschnitt des Lichtleiterendes 30 oder einer kegeligen Form des Laserdivergenzbereiches LD die Einkoppelfläche EF durch die schräge Projektion auf die Rückseite 23 elliptisch.

Die Laserachse LA trifft in einem Punkt P1 auf die Oberfläche der Rückseite 23. Der Punkt P1 liegt in etwa im Zentrum der Einkoppelfläche EF. In diesen Punkt P1 ist die Flächennormale N auf die ebene Rückseite 23 eingezeichnet, die senkrecht zur Rückseite 23 gerichtet ist. Mit dieser Flächennormale N schließt die Ausbreitungsrichtung des Laserstrahles L oder dessen Laserachse LA einen Einkoppelwinkel oder Eintrittswinkel α ein, der die Schrägstellung zwischen Laserachse LA und Rückseite 23 definiert. Der Eintrittswinkel beträgt insbesondere α = 90° - y2.

Aufgrund der Änderung der Brechzahl (oder: Brechungsindex) von der umgebenden Luft auf die höhere Brechzahl in dem Skalpellkörper 12 wird das Laserlicht L beim Eintritt oder Einkoppeln in den Skalpellkörper 12 durch die Einkoppelfläche EF gebrochen, und zwar aufgrund der Brechungsgesetze zur Flächennormalen N hin, so dass der Transmissionswinkel β zwischen der Laserachse LA und der Transmissionsrilchtung TR des Laserlichts L innerhalb des Skalpellkörpers 12 nach dem Eintritt in den Skalpellkörper 12 kleiner ist als der Eintrittswinkel α. Dabei ergibt sich der Transmissionswinkel β eindeutig durch die Brechungsgesetze aus dem Eintrittswinkel α abhängig von dem Verhältnis der meist wellenlängenabhängigen, Brechzahlen n1 der Luft einerseits und n2 des Skalpellkörpers 12 andererseits gemäß n1 sinα = n2 sinβ.

Der Eintrittswinkel α wird im Allgemeinen zwischen 15° und 55° gewählt, in einem Bereich zwischen 25° und 45°, bevorzugt in einem Bereich zwischen 30° und 40° und vorzugsweise aus einem Bereich zwischen 34° und 36°.

Es hat sich herausgestellt, dass bei den meisten kristallinen Materialien, insbesondere bei Saphir, für den Skalpellkörper 12 bei einem solchen Eintrittswinkel α zwischen der Laserachse LA und der Flächennormalen N vergleichsweise wenig Laserlicht L durch Reflektion an der Einkoppelfläche EF verloren geht, das eingestrahlte Laserlicht L also effizient ausgenutzt wird.

Als bevorzugter Wert bei der in den Figuren dargestellten Geometrie, insbesondere bei Saphir als Material für den Skalpellkörper 12, hat sich ein Eintrittswinkel α von 35° ± 1° herausgestellt, da sich damit eine vorteilhafte Leistungsverteilung der Leistung des Laserlichts L an der Skalpellschneide und dem Skalpellrücken ergibt.

Bei einem handelsüblichen Saphir ergab sich, auf eine Dezimalstelle gerundet beispielsweise bei 810 nm ein Transmissionswinkel β = 15° bei dem Eintrittswinkel α = 35° und ein Transmissionswinkel β = 17° bei dem Eintrittswinkel α = 40°, ein Transmissionswinkel β = 19° bei dem Eintrittswinkel α = 45°, ein Transmissionswinkel β = 13° bei dem Eintrittswinkel α = 30° ein Transmissionswinkel β = 11° bei dem Eintrittswinkel α = 25°.

In FIG 1 ist beispielhaft ein Transmissionswinkel β von 22° dargestellt.

Der Punkt P1, der in der Mitte der Einkoppelfläche EF liegt, liegt bevorzugt auch auf der Mittelebene M, da die Laserachse LA bevorzugt so justiert wird, dass sie in der Mittelebene M des Skalpellkörpers 12 zu liegen kommt. Das Laserlicht L wird also zentral in den Skalpellkörper 12 eingekoppelt.

Der Auftreffpunkt P1 der Laserachse LA auf der Rückseite 23 innerhalb der Einkoppelfiläche EF liegt nun um eine Höhe h1 von der Bodenfläche 24 beabstandet, und um eine Höhe h2 von dem Skalpellrücken 21 beabstandet, die größer ist als die Höhe h1. Es gilt h1 + h2 = h und h2 > h1. Das heißt, der Punkt P1 und die ganze Einkoppelfläche EF sowie die Laserachse LA liegen näher bei der Bodenfläche 24 als beim Skalpellrücken 21 oder oberhalb einer horizontalen Trennebene T des Skalpellkörpers 12, die parallel zu der Bodenfläche 24 und zum Skalpellrücken 21 zwischen diesen verläuft jeweils unter dem gleichen Abstand h/2 und die bevorzugt oder senkrecht zur Mittelebene M gerichtet ist.

Während also die Spitze 22 des Skalpellkörpers 12 auf einer Seite (in FIG 1 der Unterseite) dieser Trennebene T liegt im Abstand von h/2, liegt der Schnittpunkt P1 der Laserachse LA mit der Rückseite 23 des Skalpellkörpers 12 und ist von der Trennebene T um einen Abstand h2 - h/2 beabstandet, und liegt auch die Einkoppelfläche EF zumindest überwiegend, bevorzugt vollständig, oberhalb der Trennebene T.

Nach dem Eintritt des Laserlichts L in den Skalpellkörper 12 bei der Einkoppelfläche EF breitet sich das Laserlicht L je nach Eintrittspunkt innerhalb der Eintrittsfläche EF und der dort im Laserdivergenzbereich LD vorherrschenden Hauptausbreitungsrichtung des Laserlichts L mit einem unterschiedlichen Transmissionswinkel β in dem Skalpellkörper 12 fort. In FIG 1 sind nur für den zentralen, entlang der Laserachse LA durch den Punkt P1 verlaufenden Laserstrahl L der Transmissionswinkel β und die Transmissionsrichtung TR innerhalb des Skalpellkörpers 12 eingezeichnet.

Das Laserlicht L breitet sich aufgrund des gleichbleibenden Brechungsindex innerhalb des Skalpellkörpers 12 geradlinig fort und trifft , beispielsweise im Punkt P2 für den zentralen Laserstrahl, auf die Außenfläche des Skalpellkörpers 12 an dem Skalpellrücken 21, an der das Laserlicht L wieder auf die Luft und deren niedrigeren Brechungsindex trifft, an dem Skalpellrücken 21. Aufgrund des relativ großen Einfallswinkels δ beim Punkt P2 wird das Laserlicht L im Wesentlichen vollständig nach innen reflektiert und verbleibt aufgrund dieser internen Totalreflektion praktisch vollständig innerhalb des Skalpellkörpers 12 und verläuft nun entlang der um 180° - 2 δ gedrehten Transmissionsrichtung TR' innerhalb des Skalpellkörpers 12 weiter bis zum nächsten Außenpunkt P3, der nun schon im Schneidenbereich an der Schneidkante 20 liegt. Dort wird wieder mit dem Eintrittswinkel e, der gleich dem Ausfallswinkel ε ist, ein Teil des Laserlichts L nach innen reflektiert, jedoch tritt auch schon je nach Größe des Winkels ε bereits ein Teil aus der Schneide nach außen aus.

So setzt sich die Reflektion und Transmission des Laserlichts L im Schneidenbereich an der Vorderseite 13 in der Nähe der Spitze 22 fort, so dass im Wesentlichen das Laserlicht L an der Spitze 22 und dem sich daran anschließenden Schneidenbereich um die Schneidkante 20 und die Schneidflächen 25 und 26 nach außen in das beim Operieren oder chirurgischen Eingriff umgebende Körpergewebe nach außen austritt und eindringt. Somit vermag das Laserlicht L genau in dem Bereich, in dem mit dem Laserskalpell 2 geschnitten wird, nämlich im Bereich der Skalpellschneide, das austretende Blut zu koagulieren oder zur Gerinnung zu bringen und damit die Blutung *in situ* zu stoppen unmittelbar während des Eingriffs.

Bevorzugt wird die Mittelebene M des Skalpellkörpers 12 so gelegt bzw. der Kristall, Insbesondere Saphir, so geschliffen, dass eine Wachstumsachse oder kristallographische Vorzugsachse wie z.B. die sog. c-Achse des Kristalls, zentral in der oder parallel zur Mittelebene M liegt, insbesondere eine zentrale optische Achse des Kristalls ist, die die Schnittgerade aus der Mittelebene M und der Trennebene T ist, und parallel zum Skalpellrücken 21 zu liegen kommt, da die Transmission des Kristalls in dieser Richtung besonders hoch ist.

Die Abmessungen können (gerundet auf eine Dezimalstelle) aus folgenden bevorzugten Wertebereichen gewählt werden:
1,2 mm ≤ h ≤ 5 mm, insbesondere 2,5 mm ≤ h ≤ 3,9 mm, vorzugsweise h = 3,7 mm
0,5 mm ≤ h1 ≤ 1,6 mm, Insbesondere 0,9 mm ≤ h1 ≤ 1,3 mm, vorzugsweise h1 = 1,1 mm
0,7 mm ≤ h2 ≤ 4,5 mm, insbesondere 1,2 mm ≤ h2 ≤ 3 mm, vorzugsweise h2 = 2,6 mm
0,1 h2 ≤ h1 ≤ 0,8 h2, insbesondere 0,3 h2 < h1 ≤ 0,6 h2, vorzugsweise h1 = 0,4 h2
10 mm ≤ a ≤ 25 mm, insbesondere 15 mm ≤ a ≤ 19,5 mm, vorzugsweise a = 17,3 mm
5 mm ≤ a1 ≤ 10 mm, insbesondere 6 mm ≤ a1 ≤ 8,2 mm, vorzugsweise a1 = 7,1 mm
1,5 mm ≤ b1 ≤ 7 mm, insbesondere 2,2 mm ≤ b1 ≤ 5,5 mm, vorzugsweise b1 = 3,7 mm
1,5 mm ≤ b2 ≤ 7 mm, insbesondere 2,2 mm ≤ b2 ≤ 5,5 mm, vorzugsweise b2 = 3,7 mm
1 mm ≤ d ≤ 3,5 mm, insbesondere 1,4 mm ≤ b2 ≤ 2,8 mm, vorzugsweise d = 2,1 mm

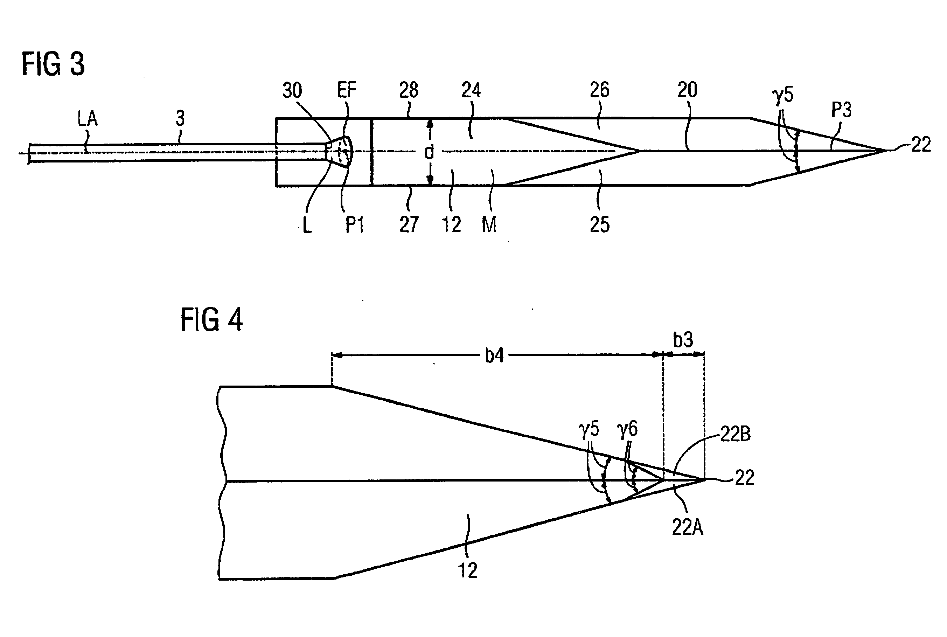

In der FIG 4 ist eine zusätzlich angeschliffene Spitze 22 mit seitlich angeschliffenen spitzen Flächen 22a und 22b, die gegenüber der ursprünglichen Spitze b3 eine Spitze, die um b3 versetzt ist, generieren. Die Länge der Spitze bis zu der neuen Spitze ist mit b4 bezeichnet und die Innenwinkel an der neuen Spitze mit γ6.

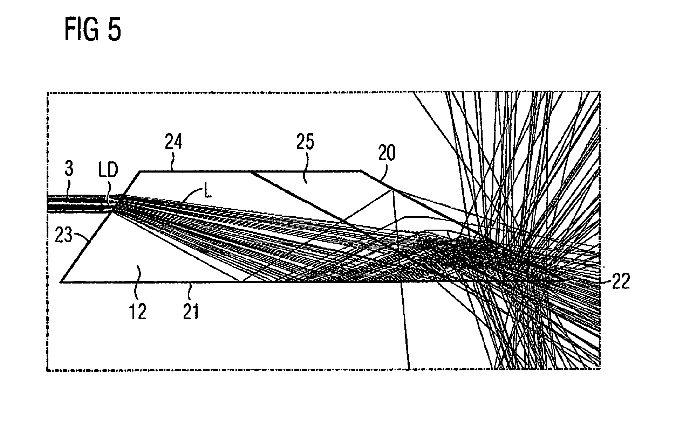

In FIG 5 ist für ein Skalpellkörper gemäß FIG 1 bis 3 ein in einer Simulation berechneter Strahlengang des Laserlichts L zu sehen. Man kann erkennen, dass der Großteil des Laserlichts L tatsächlich bis zum Schneidenbereich an der Schneidkante 20 und insbesondere im Bereich der Spitze 22 gelangt und dort aus dem Skalpellkörper 12 wieder austritt. Im Wesentlich erfolgt eine Totalreflektion an dem Skalpellrücken 21, bevor dann das Laserlicht L in den Schneidenbereich oder an die Skalpellschneide gelangt an der Vorderseite 13 des Skalpellkörpers 12. Dies ist eine Folge der außermittigen Anordnung des Lichtleichters 3 und der Einkoppelfläche EF in Bezug auf die Trennebene T. Es werden der Eintrittswinkel α der Punkt P1 mit der Einkoppelfläche EF und sein Abstand h2 von dem Skalpellrücken 21 sowie der Abstand a der Spitze 22 von der Rückseite 23 oder auch von dem Punkt P1 selbst so eingestellt, dass im Wesentlichen die eine interne Reflektion, insbesondere im oder um den Punkt P2, genügt. Der Punkt P2 ergibt sich dabei aus dem durch die Brechungsindizes und den Eintrittswinkel α festgelegten Transmissionswinkel β und der parallel zur Laserachse A verlaufenden Fläche des Skalpellrückens 21, wobei der Punkt P2 nahe genug bei der Vorderseite 13 und der Skalpellschneide, insbesondere der Spitze 22, liegt, also die Spitze 22 nicht so weit von dem Punkt P2 oder Rückseite 23 entfernt liegen soll. Üblicherweise wird zunächst der Eintrittswinkel α optimiert, um einen möglichst hohen Transmissionsanteil beim Einkoppeln des Laserlichts L an der Einkoppelfläche EF zu erhalten, und dann die Geometrie, insbesondere Länge a und Anordnung des Skalpellrückens 21 relativ zur Skalpellschneide, insbesondere Schneidkante 20 und Spitze 22, so gewählt und optimiert, dass nur eine interne Reflektion stattfindet (am Punkt P2) und der weit überwiegende Teil des Laserlichts L nach dem Durchlaufen des Skalpellkörpers 12 im Bereich der Skalpellschneide, insbesondere in der Nähe der Spitze 22, wieder aus dem Skalpellkörper 12 austritt.

### Bezugszeichenliste

- 2: Skalpell
- 3: Lichtleiter
- 12: Skalpellkörper
- 13: Vorderseite
- 20: Schneidkante
- 21: Skalpellrücken
- 22: Spitze
- 23: Rückseite
- 24: Bodenfläche
- 25, 26: Schneidenfläche
- 27, 28: Seitenfläche
- 30: Ende
- T: Trennebene
- M: Mittelebene
- N: Flächennormale
- L: Laserstrahlung
- LA: Laserachse
- LD: Laserdivergenzbereich
- EF: Einkoppelfläche
- P1, P2, P3: Punkte
- TR: Transmissionsrichtung
- α: Eintrittswinkel
- β: Transmissionswinkel
- δ, ε: Winkel
- γ1: erster Spitzenwinkel
- γ2, γ3, γ4: Innenwinkel
- γ5: zweiter Spitzenwinkel
- h, h1, h2: Höhe
- a, a1: Länge
- b1, b2: Länge
- b3, b4: Länge
- d: Breite

## Patentansprüche

1. Laserskalpell (2) mit
a) einem Skalpellkörper (12) aus einem für Laserstrahlung wenigstens eines bestimmten Wellenlängenbereiches transmissivem Material,
b) wobei der Skalpellkörper (12) eine Skalpellschneide (20) und einen Skalpellrücken (21) aufweist und die Skalpellschneide und der Skalpellrücken in einer Spitze (22) zusammenlaufen,
c) wobei der Skalpellkörper (12) ferner an einer von der Skalpellschneide abgewandten Rückseite (23) eine Einkoppelfläche (EF) zum Einkoppeln von Laserstrahlung (L) aufweist,
d) Mitteln (3) zum Einkoppeln von Laserstrahlung (L) in den Skalpellkörper (12) durch die Einkoppelfläche derer optische Achse (LA) parallel zum Skalpellrücken (21) verläuft,
e) wobei die Einkoppelfläche (EF) am Skalpellkörper (12) so angeordnet und dimensioniert ist, dass ein Teil der an der Einkoppelfläche (EF) eingekoppelten Laserstrahlung (L) an dem Skalpellrücken (21) wenigstens teilweise durch interne Reflexion zur Skalpellschneide (20) hin reflektiert wird und ein weiterer Teil der an der Einkoppelfläche (EF) eingekoppelten Laserstrahlung (L) sich auf direktem Weg durch den Skalpellkörper (12) zu dessen Skalpellschneide (20) oder dessen Spitze (22) fortpflanzt,
**dadurch gekennzeichnet, daß**
f) der zwischen der optischen Achse (LA) der Mitteln zum Einkoppeln von Lagerstrahlung und der Flächennormalen (N) der Einkoppelfläche eingeschlossene Einkoppelwinkel (α) für die Laserstrahlung (L) an der Einkoppelfläche (EF) zwischen 25° und 45° gewählt ist
g) und die Einkoppelfläche (EF) von dem Skalpellrücken (21) einen größeren Abstand (h2) aufweist als von einer Bodenfläche (24) des Skalpellkörpers (12), welche die Skalpellschneide (20) an dem von der Spitze (22) abgewandten Ende mit der Rückseite (23) des Skalpellkörpers (12) verbindet.

2. Laserskalpell nach Anspruch 1, bei der Einkoppelwinkel (α) für die Laserstrahlung (L) an der Einkoppelfläche (EF) zwischen 30° und 40° gewählt ist.

3. Laserskalpell nach Anspruch 2, bei der Einkoppelwinkel (α) für die Laserstrahlung (L) an der Einkoppelfläche (EF) zwischen 34° und 36° gewählt ist.

4. Laserskalpell nach einem der vorhergehenden Ansprüche, bei dem die Rückseite (23) des Skalpellkörpers oder zumindest die Einkoppelfläche (EF) an der Rückseite des Skalpellkörpers flach oder eben ausgebildet ist.

5. Laserskalpell nach einem der vorhergehenden Ansprüche, bei dem die Mittel zum Einkoppeln von Laserstrahlung (L) in den Skalpellkörper (12) einen Lichtleiter (3), insbesondere eine Lichtleitfaser, umfassen, dessen Ende (30) in einem Abstand zur Einkoppelfläche (EF) justiert ist.

## Claims

1. Laser scalpel (2) having
a) a scalpel body (12) of a material transmissive for laser radiation of at least one specific wavelength range,
b) wherein the scalpel body (12) has a scalpel blade (20) and a scalpel back (21) and the scalpel blade and the scalpel back converge in a point (22),
c) wherein the scalpel body (12) also has a launch surface (EF) on a rear side (23) facing away from the scalpel blade for launching laser radiation (L),
d) means (3) for launching laser radiation (L) into the scalpel body (12) through the launch surface, the optical axis (LA) of said launch surface running parallel to the scalpel back (21),
e) wherein the launch surface (EF) is disposed on the scalpel body (12) and dimensioned in such a way that a portion of the laser radiation (L) launched at the launch surface (EF) on the scalpel back (21) is reflected at least in part towards the scalpel blade (20) due to internal reflection and a further portion of the laser radiation (L) launched at the launch surface (EF) continues on a direct path through the scalpel body (12) to its scalpel blade (20) or its tip (22),
**characterised in that**
f) the launch angle (α) included between the optical axis (LA) of the means for launching laser radiation and the surface normal (N) of the launch surface for the laser radiation (L) at the launch surface (EF) is chosen between 25° and 45°
g) and the launch surface (EF) has a greater distance (h2) from the scalpel back (21) than from a base surface (24) of the scalpel body (12) which joins the scalpel blade (20) to the rear side (23) of the scalpel body (12) at the end facing away from the tip (22).

2. Laser scalpel according to claim 1, in which launch angle (α) for the laser radiation (L) at launch surface (EF) is chosen between 30° and 40°.

3. Laser scalpel according to claim 2, in which launch angle (α) for the laser radiation (L) at launch surface (EF) is chosen between 34° and 36°.

4. Laser scalpel according to one of the preceding claims, in which the rear side (23) of the scalpel body or at least the launch surface (EF) on the rear side of the scalpel body is designed to be flat or level.

5. Laser scalpel according to one of the preceding claims, in which the means for launching laser radiation (L) into the scalpel body (12) comprise a light conductor (3), in particular a fibre-optic conductor, the end (30) of which is aligned at a distance to the launch surface (EF).

## Revendications

1. Scalpel laser (2) avec
a) un corps de scalpel (12) en un matériau transmissif pour le rayonnement laser au moins d'une plage de longueurs d'onde déterminée,
b) où le corps de scalpel (12) présente une lame de scalpel (20) et un dos de scalpel (21), et la lame de scalpel et le dos de scalpel se rejoignent en une pointe (22),
c) où le corps de scalpel (12) présente en outre à un côté arrière (23) éloigné de la lame de scalpel une face de couplage (EF) pour le couplage du rayonnement laser (L),
d) des moyens (3) pour le couplage du rayonnement laser (L) dans le corps de scalpel (12) par la face de couplage, dont l'axe optique (LA) s'étend parallèlement au dos de scalpel (21),
e) où la face de couplage (EF) au corps de scalpel (12) est disposée et dimensionnée de façon qu'une partie du rayonnement laser (L) couplé à la face de couplage (EF) soit réfléchie au dos de scalpel (21) au moins partiellement par réflexion interne vers la lame de scalpel (20), et une autre partie du rayonnement laser (L) couplé à la face de couplage (EF) se propage par voie directe à travers le corps de scalpel (12) à sa lame de scalpel (20) ou sa pointe (22), **caractérisé en ce que**
f) l'angle de couplage (α) formé entre l'axe optique (LA) des moyens de couplage du rayonnement laser et la normale de face (N) de la face de couplage pour le rayonnement laser (L) à la face de couplage (EF) est sélectionné pour être entre 25° et 45°,
g) et la face de couplage (EF) présente du dos de scalpel (21) un plus grand écart (h2) qu'à partir d'une face de fond (24) du corps de scalpel (12), qui relie la lame de scalpel (20) à l'extrémité éloignée de la pointe (22) au côté arrière (23) du corps de scalpel (12).

2. Scalpel laser selon la revendication 1, dans lequel l'angle de couplage (α) pour le rayonnement laser (L) à la face de couplage (EF) est sélectionné pour être entre 30° et 40°.

3. Scalpel laser selon la revendication 2, dans lequel l'angle de couplage (α) pour le rayonnement laser (L) à la face de couplage (EF) est sélectionné pour être entre 34° et 36°.

4. Scalpel laser selon l'une des revendications précédentes, dans lequel le côté arrière (23) du corps de scalpel ou au moins la face de couplage (EF) au côté arrière du corps de scalpel est réalisé d'une manière plate ou plane.

5. Scalpel laser selon l'une des revendications précédentes, dans lequel les moyens de couplage du rayonnement laser (L) dans le corps de scalpel (12) comprennent un conducteur de lumière (3), en particulier une fibre optique, dont l'extrémité (30) est ajustée à un écart de la face de couplage (EF).
